Europäisches Patentamt

European Patent Office

Office européen des brevets

(19)

(11) Numéro de publication: **0 025 379**
**B1**

(12) **FASCICULE DE BREVET EUROPEEN**

(45) Date de publication du fascicule du brevet: **27.06.84**

(21) Numéro de dépôt: **80401205.2**

(22) Date de dépôt: **22.08.80**

(51) Int. Cl.³: **B 01 J 13/02,**
**A 01 N 25/00, A 61 K 9/50**

(54) Procédé de préparation de suspensions ou de poudres stables de microcapsules stables et d'une porosité variable et produits ainsi obtenus.

(30) Priorité: **30.08.79 FR 7921743**

(43) Date de publication de la demande:
**18.03.81 Bulletin 81/11**

(45) Mention de la délivrance du brevet:
**27.06.84 Bulletin 84/26**

(84) Etats contractants désignés:
**FR**

(56) Documents cités:
**FR - A - 2 015 022**
**FR - A - 2 192 868**
**GB - A - 929 405**
**US - A - 3 549 555**

**CHEMICAL ABSTRACTS, vol. 87, no. 22, 28**
**novembre 1977, réf. 172793y page 305**
**Columbus, Ohio, US A. PALMIERI: "Production**
**of a coacervate film for microcapsule diffusion**
**studies"**

(73) Titulaire: **ROUSSEL-UCLAF**
**35, boulevard des Invalides**
**F-75007 Paris (FR)**

(72) Inventeur: **Suglia, Jean-Claude**
**"Le Deven" no 13**
**F-13112 La Destrousse (FR)**
Inventeur: **Meinard, Colette**
**24, Avenue Petite Suisse**
**F-13012 Marseille (FR)**

(74) Mandataire: **Markovic, Borivoj et al,**
**102, route de Noisy Boîte Postale no 9**
**F-93230-Romainville (FR)**

Courier Press, Leamington Spa, England.

## Description

La présente invention a pour objet un procédé de préparation de suspensions ou de poudres stables de microcapsules stables et de porosité variable et les suspension ou les poudres obtenues par ce procédé.

L'invention a plus particulièrement pour objet un procédé de préparation de suspension ou de poudres stables de microcapsules stables dotées d'une porosité variable contenant une ou plusieurs matières actives.

Par le terme de matière active, on entend dans ce qui suit toute matière encapsulée selon la présente invention apte à agir sur son environnement.

Les microcapsules obtenues au cours de l'exécution du procédé de l'invention présentent une stabilité face aux influences physicochimiques et plus généralement aux éléments extérieures, tels que rayonnement lumineux ou changements de la température. Elles assurent aussi une protection efficace des matières actives microencapsulés et leur permettent de garder leur activité biologique.

Le procédé de l'invention permet en outre l'obtention de microcapsules de porosité variable selon le besoin de l'utilisation. En effet, grâce à ce procédé la porosité de la paroi de microcapsules peut être controlée selon les bsoins de l'utilisation.

Les suspensions et les poudres, également objet de l'invention, obtenues à partir des microcapsules réalisées lors de l'exécution du procédé, présentent également une stabilité remarquable lors de leur stockage ainsi qu'une résistance aux influences chimiques et aux éléments extérieures, tels que le rayonnement lumieux, le changement de température ou la résistance à l'écrasement.

On connaissait déjà des procédés de préparation de suspensions ou de poudres de microcapsules contenant une matière active dont l'enveloppe était obtenue par coacervation de solutions colloïdales et encapsulation consécutive des gouttelettes émulsifiées, telles que celles de gélatine et de gomme d'acacia.

Selon ces procédés, on préparait à chaud et sous agitation une solution colloïdale aqueuse de gélatine et de gomme d'acacia, puis additionnait la ou les matières actives sous forme d'une solution, d'une émulsion ou d'une dispersion huileuse.

On soumettait ensuit l'émulsion résultante à la coacervation et à l'encapsulation consécutive de la matière active présente par ajustement du pH au moyen d'un acide ou d'une base par dilution et par abaissement de la température du milieu réactionnel.

Une fois les microcapsules formées, on procédait à la réticulation du coacervat formant la paroi de l'enveloppe des gouttelettes encapsulées. La réticulation s'effectuait soit par un aldéhyde soit par un dérivé de la famille du tanin.

On procédait ensuite à la mise en suspension des microcapsules obtenues ou les isolait sous forme de poudres.

La qualité de la paroi de l'enveloppe formant une telle microcapsule pouvait varier au point de vue de la perméabilité.

Cette perméabilité étant fonction de la porosité de la paroi, dépendait de différents facteurs. Parmi ces facteurs figuraient notamment la vitesse et le degré d'abaissement de la température du milieu réactionnel lors de la coacervation, la quantité du tanin utilisé pour la réticulation du coacervat, les propriétés physiques du ou des produits microencapsulés (viscosité, volatilité, l'état solide ou l'état liquide), l'épaisseur de la paroi de l'enveloppe ou la grosseur des particules à enrober. Pour une même quantité de produit microencapsulé la surface d'échange était plus importante avec des microcapsules à base de petites particules qu'à base de grosses particules.

Lors de l'exécution de ces procédés, afin d'obtenir des microcapsules d'une taille raisonnablement petite, on était obligé de recourir à des vitesses d'agitation assez élevées. Ce fait représentait à l'échelle industrielle un inconvénient du fait de formation de mousse lors de l'agitation et d'autre part exigeait des installations coûteuses.

Les quantités globales de collaïdes utilisées lors de l'exécution de ces procédés étaient assez élevées et donnaient lieu à la formation de microcapsules qui souvent n'avient pas les qualités requises. Ainsi, par exemple, les parois de l'enveloppe formée devenaient très denses, d'où l'impossibilité d'effectuer correctement la réticulation du coacervat en profondeur. Il en résultait ainsi que l'insolubilité et la résistance aux effets extérieurs physiques et chimiques ne pouvaient pas être toujours réalisées comme souhaité.

Ces faits prennent de l'importance lors de l'utilisation de telles microcapsules en agriculture par exemple. La durée de vie des microcapsules contenant une matière active, dans ce cas, doit être suffissante pour assurer l'activité maximum de la matière contenue.

D'autre part, les suspensions à base des microcapsules obtenues par ces procédés ne supportaient pas toujours un long stockage par exemple. En effet, souvent chez de telles suspensions, se produisait après un certain temps, une sédimentation des microcapsules et apparaissait un phénomène d'agglomération. Une telle suspension lors de la dilution pour la pulvérisation par exemple, risquait de n'être plus homogène et de boucher l'orifice de pulvérisation.

En ce qui concerne les poudres réalisées à partir des microcapsules obtenues par ces procédés,

2

étant donné que les microcapsules tendaient souvent à s'agglomérer et à coller lors du séchage, on était obliqué dans un tel cas de recourir au broyage afin d'obtenir une poudre finement divisée.

Afin de remédier à ces inconvénients la Société demanderesse a mis au point le procédé de la présente invention.

Ce procédé permet notamment l'obtention de microcapsules, contenant une ou plusieurs matières actives, dont la porosité des parois peut être controlée selon le besoin. Les microcapsules obtenues par ce procédé sont dotées également d'une stabilité face aux influences chimiques et aux éléments extérieurs. Il en est de même pour les suspensions ou les poudres de ces microcapsules qui sont aptes à être stockées à volonté.

Ces résultats ont pu être obtenus grâce au choix particulier des paramètres, tels que la concentration totale en colloïdes, la présence d'un moyen de controle de porosité, la vitesse d'agitation, le choix du pH du milieu réactionnel, le rapport des concentrations des colloïdes et de la ou des matières actives à encapsuler, la quantité du solvant organique, la durée de coacervation par l'abaissement de la température et le choix de la température de coacervation et de la microencapsulation consécutive, la manière d'effectuer la réticulation du coacervat en deux stades et la façon de réaliser les suspensions et les poudres desdites microcapsules. Comme la concentration totale en colloïdes a une grande incidence sur le volume de coacervat à obtenir et comme la microencapsulation est une adsorption superficielle du coacervat sur la surface des gouttelettes émulsionnées dans la phase aqueuse, lors de la mise au point du présent procédé une attention particulière a été consacrée au choix de la concentration en colloïdes par rapport à la concentration de la ou des matières actives et à la quantité de solvant organique.

Grâce à ce choix judicieux, le présent procédé permet la formation du coacervat en une quantité suffisante pour l'enrobage de la surface globale des gouttelettes présentes dans l'émulsion "huile dans l'eau" par une couche de coacervat de qualité recherchée.

Parmis les qualités recherchées de la couche du coacervat constituant la paroi de la microcapsule enrobant une gouttelette émulsifiée, la perméabilité joue un rôle important. Cette perméabilité étant fonction de la porosité de ladite paroi, le procédé de l'invention se proposait de trouver un moyen de la contrôler.

En effet il a été trouvé par la Société demanderesse que, au lieu de chercher à déterminer à chaque occasion les nombreux paramètres gouvernant une microencapsulation, au moyen de la gélatine et de la gomme d'acacia, afin de produire des parois d'une porosité déterminée comme l'art antérieur l'enseigne, il était avantageux de procéder de la manière suivant:

Lors du premier stade, on détermine une fois pour toutes les paramètres optimaux d'une telle microencapsulation en fonction de la porosité maximale voulue, puis ces paramètres établis, on procède au contrôle de la porosité des parois proprement dit. Ce but est atteint par l'introduction dans l'emulsion destinée à la coacervation d'une quantité variable d'un produit susceptible d'être occlus par le coacervat. L'occlusion de ce produit provoque l'obturation des pores des parois formées par le coacervat. Selon la quantité utilisée de ce produit, les pores seront plus ou moins obturés et de ce fait on pourra contrôler la porosité des microcapsules.

Il n'est pas nécessaire de souligner l'importance de la porosité et de son contrôle dans les cas de microcapsules contenant une matière active. Grâce à cette porosité, les matières actives peuvent être diffusées plus ou moins longtemps selon l'ordre de grandeur des pores de la paroi et la nature de ladite matière. En connaissant la durée recherchée de la diffusion d'une matière active, on peut donc produire les microcapsules dotées d'une porosité qui assurera cette diffusion.

Pour satisfaire à l'exigence de la résistance aux influences chimiques et plus généralement aux éléments extérieurs, tels que le rayonnement lumineux, le changement de la température, la solubilité ou la résistance mécanique, les conditions de la coacervation et de la microencapsulation étant déterminées, il a été trouve que cette résistance peut être notablement améliorée, si la réticulation du coacervat est exécutée en deux stades.

En effet, dans un premier stade en faisant agir sur le coacervat un aldéhyde, celui-ci réagit avec une partie des groupes aminés de la gélatine et insolubilise les microcapsules formées. Dans un deuxième stade on complète la réticulation en faisant agir le tanin qui réagit aussi avec les fonctions aminées encore libres de la gélatine. De cette manière, s'établissement également des liaisons transversales entre les molécules de gélatine et une microcapsule acquiert les qualités de stabilité recherchées.

Les microcapsules obtenues selon le procédé de la présente demande, peuvent contenir, comme produits encapsulés, divers agents pesticides tels que biocides, fongicides, insecticides herbicides, acaricides, nématicides, ainsi que des virus, des enzymes, des phéromones, des hormones juvéniles ou encore des milieux nutritifs artificiels attractifs.

Les microcapsules obtenues selon le procédé de la présente demande peuvent contenir un seul des agents nommés ci-dessus ou plusieurs de ces agents en association. Elles peuvent contenir, par exemple, un insecticide associé à une phéromone, une hormone juvénile ou un virus.

Il est bien évident que l'on n'associe, le cas échant, dans une même microcapsule que deux ou plusieurs agents chimiquement compatibles. Quand ce n'est pas le cas, on procède à des microcapsulations séparées, le mélange intervenant ultérieurement.

## 0 025 379

Les microcapsules obtenues selon le procédé de l'invention peuvent être utilisées notamment dans l'agriculture, mais, par ailleurs, elles peuvent faibre l'objet d'autres autilisations, par exemple, dans les peintures et les pâtes à papier, pour améliorer la persistance de l'action d'un principe actif ou encore dans les aérosols afin d'éviter lors de la pulvérisation une trop grande adsorption du produit par le support.

Parmi les utilisations possibles, on peut citer plus précisément la lutte biologique (par exemple simulation d'oeufs artificiels pour la production de trichogrammes), les formulations à effets progressif, les présentations possibles pour les attractifs ou répulsifs à durée d'action prolongée, les traitement des grandes étendues (étangs, forêts...), les traitements des étables, la microencapsulation des produits aqueux, la microencapsulation de matières actives incompatibles.

Il peut également être intéressant d'associer certaines microcapsules dans la mesure où elles ne renfermant pas déjà une association. One peut ainsi associer, par exemple, des microcapsules renfermant un pesticide, notamment un pyréthrinoïde et des microcapsules renfermant une phéromone, une hormone juvénile ou un virus.

Dans le cas d'une utilisation comme insecticide, les microcapsules obtenues selon le procédé de l'invention peuvent être conçues soit pour libérer le principe actif dans l'atmosphère, soit pour être ingérées par les insectes, le principe actif étant dans ce cas libéré à l'intérieur de l'organisme de l'insecte.

Le procédé, objet de la présente invention, concerne donc la préparation de suspensions ou de poudres stables de microcapsules stables et d'une porosité variable, contenant au moins une matière active, procédé comportant les stades:

— de préparation d'une émulsion "huile dans l'eau" à partir d'une solution colloïdale de gélatine et de gomme d'acacia à poids égal, leur concentration totale allant de 4 à 6% du poids d'émulsion et d'une émulsion, d'une solution ou d'une dispersion huileuse de la ou des matières actives, la concentration allant de 1 à 5% du poids de l'émulsion "huile dans l'eau", sous agitation, à une température proche de 50°C et en présence d'un moyen émulsionnant,

— de coacervation et de microencapsulation consécutive de gouttelettes émulsionnées, contenant la ou les matières actives, par ajustement de la valeur du pH du milieu réactionnel à 4,2—4,4 au moyen d'un acide, puis par abaissement de la température vers 20°C toujours sous agitation,

— de réticulation des parois de microcapsules formées, par action d'un moyen susceptible de réagir avec les fonctions aminées de la gélatine,

— et de formulation des microcapsules obtenues, procédé caractérisé en ce que:

— l'on ajoute à ladite émulsion "huile dans l'eau" une quantité d'éthyl hydroxyéthylcellulose organosoluble variable selon la porosité désirée des microcapsules, celle-ci étant d'autant plus petite que la quantité d'éthylhydroxyéthyl cellulose organosoluble est plus forte,

— l'on fait agir sur les microcapsules formées à la suite de la coacervation, successivement, comme moyen de réticulation, de l'aldéhyde glutarique et du tanin, sous agitation lente à 20°C et obtient les microcapsules réticulées que soit l'on additionne, sous agitation lente, à 20°C d'éthyl hydroxyéthylcellulose hydrosoluble et obtient la suspension concentrée de microcapsules, soit l'on traite par un véhicule solide sous agitation à 20°C et isole après séchage, la poudre cherchée.

Ce procédé est encore caractérisé par les points suivants:

— on prépare l'émulsion "huile dans l'eau" par agitation d'environ 400 à 600 t/mn en présence d'un agent anti-mousse, tel qu'un alcool secondaire comme l'alcool octylique secondaire, dont la concentration est de 0,2 à 0,7% du poids de ladite émulsion et en présence d'un mélange d'un agent tensio-actif anionique et d'un agent tensio-actif non ionique,

— la concentration d'éthyl hydroxyéthylcellulose organosoluble dans l'émulsion "huile dans l'eau" est de 0,01 à 20% du poids des colloïdes,

— la concentration de l'aldéhyde glutarique est de 2 à 5% et celle du tanin est de 3 à 8% du poids de colloïdes et l'agitation, à ce stade, est d'environ 400 à 600 t/mn à 20°C;

— on ajoute à la suspension des microcapsules réticulées 0,5 à 2% en poids d'éthyl hydroxyéthylcellulose hydrosoluble;

— on ajoute à la suspension des microcapsules réticulées, 1 à 15% en poids de chlorure de calcium;

— on ajoute l'éthyl hydroxyéthylcellulose hydrosoluble et le chlorure de calcium en présence d'un agent tensio-actif non-ionique, sous agitation de 400 à 600 t/mn à 20°C;

— le véhicule solide par lequel on triate les microcapsules réticulées est le talc, à une concentration pondérale de 7 à 13% de la suspension desdites microcapsules;

— la matière active à microencapsuler est un dérivé pyréthrinoïde, notamment le (1R,cis)2,2-diméthyl 3-(2,2-dibromovinyl)cyclopropane-1-carboxylate de (S)$\alpha$-cyano 3-phénoxy benzyle ou décaméthrine ou le (1R,3S,E)2,2-diméthyl 3-(2-oxo 2,3,4,5-tétrahydro 3-thiophénylidène méthyl)cyclopropane-1-carboxylate de 5-benzyl 3-furylméthyle ou kadéthrine;

— la matière active à microencapsuler est un virus notamment,

4

# 0 025 379

le Bacillus Thurigiensis ou le Heliothis Nuclear Polyhedrosis Virus", un enzyme, une phéromone, une hormone juvénile, un milieu nutrific artificiel, un insecticide non pyréthrinoïde, un herbicide, un biocide, un fongicide, un acaricide ou un nématicide, ou encore une association de deux ou plusieurs agents mentionnés ci-dessus.

La présente invention couvre:

— les suspensions ou les poudres stables à base de microcapsules stables contenant une matière active obtenues selon le procédé de l'invention;
— les suspensions ou les poudres stables obtenues selon le procédé de l'invention, la matière active étant un dérive pyréthrinoïde, notamment le (1R,cis)2,2-diméthyl 3-(2,2-dibromovinyl)cyclopropane-1-carboxylate de (S)$\alpha$-cyano 3-phénoxy benzyle ou décaméthrine ou le (1R,3S,3E)2,2-diméthyl 3-(2-oxo 2,3,4,5-tétrahydro 3-thiophénylidène méthyl) cyclopropane-1-carboxylate de 5-benzyle 3-furylméthyle ou kadéthrine,
— les suspensions ou les poudres stables obtenues selon le procédé de l'invention, la matière active étant un virus, notamment le Bacillus Thurigiensis ou l'"'Heliothis Nuclear Polyhedrosis Virus",
— les suspensions ou les poudres stables obtenues selon le procédé de l'invention, la matière active étant un enzyme, une phéromone, une hormone juvénile, un milieu nutritif artificiel, un insecticide non pyréthrinoïde, un herbicide, un biocide, un fongicide, un acaricide, un nématicide ou une association de deux ou plusieurs agents mentionnés ci-dessus.

Le procédé de l'invention peut encore être illustré par les données exposées ci-après:

— La concentration totale en colloïdes est choisie de manière à être de préférence de 5%, afin de donner dans des conditions réactionnelles choisies, le volume maximal du coacervat.
— La matière active à microencapsuler peut être liquide ou solide mais n'est pas hydrosoluble. Elle est mise en solution, émulsion ou dispersion dans un solvant organique usual tel que le xylène, le diméthylphtalate ou un autre phtalate. Une telle solution, émulsion ou dispersion peut être préparée en utilisant un agent tensio-actif non ionique, tel que le Galoryl EM 60® et un agent tensio-actic anionique tel que le Galoryl 520®. La nature des agents tensio-actifs ainsi que leur concentration sont choisis de manière à ne pas perturber la coacervation et la microencapsulation.
— L'acide utilisé pour l'ajustement de la valeur du pH est l'acide acétique à 10%.
— Lors de la préparation d'une suspension concentrée, à l'aide des microcapsules obtenues comme décrit ci-dessus, il faut tenir compte de facteurs comme la protection contre le gel, la viscosité de la suspension et la tenue en suspension lors de la dilution pour son utilisation.
— On utilise comme moyen antigel le chlorure de calcium, par exemple, à une concentration de 12%:
— Le choix de l'épaississeur au moyen duquel on ajuste la viscosité de la suspension dépend de son inertie chimique vis-a-vis des différents constituants d'une suspension. Dans le cas présent on choisit notamment l'éthyl hydroxyéthylcellulose hydrosoluble. Du fait de la concentration choisie du chlorure de calcium, on évite que la floculation de l'éthylhydroxyéthylcellulose puisse apparaître.

Dans le cas de préparation de poudres à base des microcapsules obtenues selon le procédé de l'invention, on ajoute sous agitation au mélange réactionnel, une fois la réticulation du coacervat accomplie, du talc à une concentration en poids de 10% environ. Le talc enrobe les microcapsules réticulées et empêche ainsi qu'elles s'agglomèrent lors du sèchage. De cette manière on évite ensuite de recourir au broyage des microcapsules agglomérées afin d'obtenir une poudre fine. La suppression du broyage représente aussi un avantage important du procédé de l'invention, du fait que les parois des microcapsules ne risquent pas d'être endommagées. L'intégrité des microcapsules étant donc maintenue, la diffusion envisagée de la matière active contenue ne risque donc pas d'être modifiée.

Il va être donné maintenant à titre non limitatif des exemples de mise en oeuvre du procédé de l'invention.

Example 1:
Préparation d'une suspension aqueuse de microcapsules à faible porosité contenant le 1R, cis-2,2-diméthyl 3-(2,2-dibromovinyl) cyclopropane-1-carboxylate de (S)$\alpha$-cyano 3-phénoxybenzyle ou décaméthrine (produit A).

On prépare sous agitation de 500 tours/minute à 50°C une première solution (I) de 20 g de gélatine et de 20 g de gomme d'acacia dans 760 ml d'eau déminéralisée en présence de 0,5 g d'octanol secondaire.

On prépare d'autre part une deuxième solution (II) sous agitation de 500 tours/minute et à 50°C, à partir de 25 g de composé A, 46,4 g de xylène, 96,6 de diméthyl phtalate, 4,0 g d'octanol secondaire, 6,5 g d'éthyl hydroxyéthylcellulose organosoluble, 0,8 g de Galoryl EM 520 et 0,2 g de Galoryl EM 60.

On prépare l'émulsion, "huile dans l'eau" en introduisant lentement la solution II dans la solution I, toujours sous agitation de 500 tours/minute à 50°C.

Une fois l'émulsion "huile dans l'eau" réalisée, on ajuste le pH du milieu réactionnel entre 4,2 et

5

4,4 par addition d'une solution d'acide acétique à 10%, puis abaisse lentement, en une heure environ, la température à 20°C.

On ajoute ensuite, toujours sous agitation de 500 tours/minute, 5 ml d'aldéhyde glutarique à 25%. Après une heure environ, toujours sous agitation de 500 tours/minute, on ajoute 10 g d'une solution aqueuse de tanin à 15%. Le mélange réactionnel est maintenu sous agitation de 500 tours/minute, à température ambiante pendant 3 heures environ. On obtient ainsi des microcapsules réticulées, dont le diamètre est au plus égal à 30 $\mu$m.

Une fois les microcapsules réticulées formées, on ajoute successivement 1 g d'octanol secondaire, 119 g de chlorure de calcium (par petites fractions), 10 g d'éthyl hydroxyéthylcellulose hydrosoluble, 12 g de Galoryl EM 42 et 0,04 g de Rhodamine B, puis continue l'agitation pendant environ 3 heures et passe la suspension obtenue sur un tamis. Cette suspension contient 98,1% du principe actif utilisé au départ.

Example 2:

Préparation d'une suspension aqueuse de microcapsules à forte porosité contenant le composé A.

De façon analogue à celle de l'exemple 1 et en utilisant les mêmes quantités des ingrédients, mais en supprimant la présence d'éthyl hydroxyéthycellulose organosoluble dans la solution II, on obtient la suspension dont les microcapsules sont dotées d'une forte porosité. Cette suspension contient 98,7% du principe actif utilisé au départ.

Exemple 3:

Préparation d'une poudre à base des microcapsules.

Les microcapsules obtenues selon les exemples 1 et 2 peuvent être isolées aussi sous forme d'une poudre. Pour préparer une telle poudre, on ajoute sous agitation de 500 tours/minute à l'ensemble du mélange réactionnel obtenu après la réticulation du coacervat de l'exemple 1 ou 2, 110 g de talc, maintient l'agitation pendant une quinzaine de minutes environ, fait passer la suspension par un tamis, essore les microcapsules et les sèche. La poudre obtenue peut ensuite être conditionnée selon les méthodes habituelles, comme par exemple sous forme d'appâts.

Exemple 4:

Préparation d'une poudre à base de microcapsules contenant un virus "Heliothis Nuclear Polyhedrosis"

On prépare sous agitation de 500 tours/minutes à 50°C une première solution (I) de 20 g de gélatine et de 20 g de gomme d'acacia dans 760 ml d'eau déminéralisée.

On prépare d'autre part une deuxième solution (II) sous agitation de 500 tours/minute et à 50°C à partir de 25 g de poudre de virus "Heliothis Nuclear Polyhedrosis" 46,4 g de xylène, 96,6 g de diméthyl phtalate, 0,8 g de Galoryl EM 520 et 0,2 g de Glaroyl EM 60.

On prépare l'émulsion "huile dans l'eau" en introduisant lentement la solution II dans la solution I toujours sous agitation de 500 tours/minute à 50°C.

Une fois l'émulsion "huile dans l'eau" réalisée, on ajuste le pH du milieu réactionnel entre 4,2 et 4,4 par addition d'une solution d'acide acétique à 10% puis abaisse lentement en une heure environ, la température à 20°C.

On ajoute ensuite toujours sous agitation de 500 tours/minute, 5 ml d'aldéhyde glutarique à 25%. Après une heure environ, toujours sous agitation de 500 tours/minute, on ajoute 10 g d'une solution aqueuse de tanin à 15%. Le mélange réactionnel est maintenu sous agitation de 500 tours/minute, à température ambiante pendant 3 heures environ. On obtient ainsi des microcapsules réticulées, dont le diamère est au plus égal à 30 $\mu$m.

Une fois les microcapsules réticulées formées, on ajoute sous agitation de 500 tours/minute à l'ensemble du mélange réactionnel obtenu après la réticulation du coacervat, 110 g de talc, maintient l'agitation pendant une quinzaine de minutes environ, fiat passer la suspension par un tamis, essore les microcapsules et les sèche. La poudre obtenue peut ensuite être conditionnée selon les méthodes habituelles, comme, par exemple, sous forme d'appâts.

Il est remarquable que le procédé de l'invention permette la microencapsulation de virus sans les détruire.

Example 5:

Etude de porosité des microcapsules obtenues selon le procédé de l'invention.

Pour étudier la porosité des microcapsules, on procède à leur préparation selon la méthode décrite aux exemples 1 et 2, tout en choisissant que la matière microencapsulée soit le naphtalène au lieu du composé A.

La concentration de l'éthyl hydroéthylcellulose organosoluble de la solution I a varié selon les essais, comme indiqué au tableau suivant:

**0 025 379**

|  | Essais A | Essais B | Essais C |
|---|---|---|---|
| Naphtalène | 25% | 25% | 25% |
| Xylène | 75% | 70% | 65% |
| Ethyl hydroxyéthylcellulose organosoluble | 0 | 5% | 10% |

100 mg des microcapsules obtenues contenant du naphtalène provenant des essais A, B et C, sont déposés sur des rondelles de papier filtre. On place comme référence une quantité équivalente de naphtalène non microencapsulé. L'étude est faite à l'abri de la lumière, à une température et une humidité relative moyennes égales respectivement à 25°C et 60%, sous un courant d'air de 143 m3/heure.

Le dosage du naphtalène sublimé est effectué sur un appareil de chromatographie en phase vapeur et après 24 heures, 7 jours et 28 jours.

Le naphtalène non microencapsulé est totalement sublimé après 24 heures, tandis que les microcapsules des essais A, B et C ont donné les pourcentages suivants de pertes du naphtalène en fonction du temps et de la quantité d'éthyl hydroxyéthylcellulose organosoluble (EHEC) contenue dans les parois des microcapsules:

|  | Sublimitation % | | |
|---|---|---|---|
|  | 1 jour (24 h) | 7 jours | 28 jours |
| Naphtalène non microcapsulé | 100% | — | — |
| Microcapsules à 0% d'EHEC | 22% | 28% | 30% |
| Microcapsules à 5% d'EHEC | 16% | 22% | 24% |
| Microcapsules à 10% d'EHEC | 0% | 0% | 0% |

One peut constater d'après ces essais que la différence de porosité des microcapsules dépend de la concentration en éthyl hydroxyéthylcellulose organosoluble dans les parois.

En variant donc la concentration d'éthyl hydroxyéthylcellulose organosoluble contenue dans les microcapsules, il est possible de contrôler la porosité de la paroi d'une microcapsules en fonction de la nature de la matière active à encapsuler et de l'usage auquel elle est destinée.

Example 6:
Etude de la protection contre le rayonnement lumineux fournie par les microcapsules réalisées selon l'invention à une matière active.

Pour effectuer cette étude, un composé photosensible, comme par exemple le (1R,3S,E) 2,2-diméthyl 3-(2-oxo 2,3,4,5 tétrahydro 3-thiophenylidène méthyl) cyclopropane carboxylate de 5-benzyl-3-furylméthyle ou kadéthrine (composé B), a été microencapsulé et les microcapsules mises en suspension selon le procédé de l'invention. Cette suspension et un concentré émulsifiable à base du même composé photosensible et à la même concentration, ont été exposés au même rayonnement lumineux, reproduisant le spectre solaire. La température et l'humidité relative moyennes ont été respectivement de 25°C et de 75%.

Les dosages des pertes de matière active (composé B) ont été effectuées après 24 heures, 7 et 14 jours d'exposition audit rayonnement, et au moyen d'un appareil de chromatographie en phase liquide à haute pression.

Les résultats de ces essais sont donnés dans le tableau suivant:

| Temps | 24 heures | 7 jours | 14 jours |
|---|---|---|---|
| % de perte de concentré émulsifiable | 100% | 100% | 100% |
| % de perte de suspension de microcapsules | 13% | 54% | 71% |

7

Ces résultats démontrent bien l'efficacité de la protection de la matière active microencapsulée selon le procédé de l'invention.

Exemple 7:
Etude de conservation d'activité biologique d'une matière active microencapsulée selon le procédé de l'invention.

Pour effectuer cette étude, le composé A (Décaméthrine) a été microencapsulé et mis en suspension ou conditionné sous forme de granulés préparés à partir de la poudre desdites microcapsules. Cette suspension et ces granulés ont été comparés à un concentré émulsifiable à base dudit produit A à une concentration identique.

a) Etude d'activité biologique de la suspension des microcapsules et du concentré émulsifiable.

L'insecte utilisé pour ce test biologique est une chenille (Spodoptera Littoralis). On dilue à l'eau la suspension des microcapsules et le concentré émulsifiable, les deux dilutions aqueuses contenant le produit A sont ensuite pulvérisées à raison de 10 ml pour 4 plants de haricots (8 feuilles). Il est déposé sur chaque plant 0, 3, 5, 7 et 14 jours après le traitement vingt chenilles (au stade L 4). Les contrôles sont effectués 24 et 48 heures après l'infestation.

Les résultats de ces essais sont donnés dans le tableau suivant:

| Traitements | Doses de matière active g/hl | Pourcentage de mortalité aux infestations à: | | | | | | | | | |
|---|---|---|---|---|---|---|---|---|---|---|---|
| | | J=0 | | 3 jours | | 5 jours | | 7 jours | | 14 jours | |
| | | 24 h | 48 h | 24 h | 48 h | 24 h | 48 h | 24 h | 48 h | 24 h | 48 h |
| Suspension aqueuse fluide de microcapsules | 10 | 95 | 100 | 90 | 90 | 75 | 80 | 65 | 70 | 30 | 45 |
| concentré émulsifiable | 10 | 100 | 100 | 5 | 15 | 5 | 5 | 0 | 10 | 5 | 10 |

## 0 025 379

Ces résultats (tableau ci-dessus) indiquent que le concentré émulsifiable a une baisse d'activité très nette après le troisième jour; alors que la suspension de microcapsules possède encore une certaine activité après quatorze jours.

b) Etude d'activité biologique d'une matière active microencapsulée suivant l'invention et conditionnée sous forme d'appâts en granulés.

Ces appâts en granulé sont obtenus à partir des microcapsules isolées, sous forme d'une poudre et contenant le produit A comme matière active.

Ils ont été répandus sur le sol pour protéger des laitues contre l'attaque des chenilles (Spodoptera Littoralis). Au préalable, il a été constaté que le produit A mis dans des appâts directement, sans être microencapsulé, se dégrade très rapidement et perdait vite son activité biologique.

La parcelle traitée contient vingt laitues au stade 2—3 feuilles repiquées suivant quatre lignes.

On dépose au sol, vingt chenilles. Les contrôles ont eu lieu dix neuf jours après la contamination. Les résultats (tableau ci-dessous) montrent que les appâts présentent un niveau d'activé tout à fait comparable à un produit de référence (Toxaphène).

| Dose en g de matière active par ha | Mode de traitement | Pourcentage de laitues sectionnées | Pourcentage de chenilles vivantes |
|---|---|---|---|
| Composé A 500 g/ha (microcapsules) | Epandage à la surface du sol | 0% | 4% |
| Toxaphène 100 g/ha (granulés) | Epandage à la surface du sol | 27,5% | 66,2% |

Les résultats de ces études démontrent clairement que grace au procédé de l'invention, il est possible de réaliser les suspensions ou les poudres stables à base de microcapsules stables, contenant des matières activés, assurant le contrôle de diffusion desdites matières actives selon leur nature et selon les exigences d'utilisation. En outre, ils démontrent que les microcapsules fournissent à une matière encapsulée une protection contre les influences chimiques et les éléments extérieures, tels que le rayonnement lumineux ou le changement de la température, tout en conservant l'activité biologique de ladite matière.

Comme on peut le constater, lors des études dont les résultats viennent d'être présentés, ont été cités des produits utilisés habituellement dans le domaine agricole.

Néanmoins, toute autre matière active destinée à l'utilisation alimentaire, diététique, diagnostique, pharmaceutique ou parapharmaceutique ainsi que toute autre matière active dont les utilisations possibles, autres que celles mentionnées ci-dessus, ont été décrites précédemment, peuvent être microencapsulées selon le procédé de l'invention et le microcapsules ainsi obtenues peuvent être conditionnées sous les formes habituelles correspondant à utilisation.

**Revendications**

1. Procédé de préparation de suspensions ou de poudres stables de microcapsules stables et d'une porosité variable contenant au moins une matière active, procédé comportant les stades:

— de préparation d'une émulsion "huile dans l'eau" à partir d'une solution colloïdale de gélatine et de gomme d'acacia à poids égal, leur concentration totale allant de 4 à 6% du poids d'émulsion et d'une émulsion, d'une solution ou d'une dispersion huileuse de la ou des matières actives, la concentration allant de 1 à 5% du poids de l'émulsion "huile dans l'eau" sous agitation à une température proche de 50°C et en présence d'un moyen émulsionnant,

— de coacervation et de microencapsulation consécutive de gouttelettes émulsionnées contenant la ou les matières actives par ajustement de la valeur du pH du milieu réactionnel à 4,2—4,4 au moyen d'un acide puis par abaissement de la température vers 20°C toujours sous agitation,

— de réticulation des parois de microcapsules formées par action d'un moyen susceptible de réagir avec les fonctions aminées de la gélatine,

— et de formulation des microcapsules obtenues, procédé caractérisé en ce que:

— l'on ajoute à ladite émulsion "huile dans l'eau" une quantité d'éthyl hydroxyéthylcellolose organosoluble variable selon la porosité désirée des microcapsules, celle-ci étant d'autant plus petite que la quantité d'éthylhydroxyéthyl cellulose organosoluble est plus forte,

— l'on fait agir sur les microcapsules formées à la suite de la coacervation successivement, comme moyen de réticulation, de l'aldéhyde glutarique et du tanin sous agitation lente à 20°C et obtient les microcapsules réticulées que:

**0025379**

soit l'on additionne sous agitation lente à 20°C, d'éthyl hydroxyéthylcellulose hydrosoluble et obtient la suspension concentrée de microcapsules,
soit l'on traite par un véhicule solide, sous agitation à 20°C et isole après séchage la poudre cherchée.

2. Procédé selon la revendication 1, caractérisé en ce que l'on prépare l'émulsion "huile dans l'eau" par agitation d'environ 400 à 600 tours/minute, en présence d'un agent anti-mousse, tel qu'un alcool secondaire, comme l'alcool octylique secondaire, dont la concentration est de 0,2 à 0,7% du poids de ladite émulsion et en présence d'un mélange d'un agent tensio-actif anionique et d'un agent tensio-actif non ionique.

3. Procédé selon la revendication 1 ou 2, caractérisé en ce que la concentration d'éthyl hydroxyéthylcellulose organosoluble dans l'émulsion "huile dans l'eau" est de 0,01 à 20% du poids des colloïdes.

4. Procédé selon l'une quelconque des revendications 1 à 3, caractérisé en ce que la concentration de l'aldéhyde glutarique est de 2 à 5% et celle du tanin est de 3 à 8% du poids des colloïdes et l'agitation, à ce stade, est d'environ 400 à 600 tours/minute à 20°C.

5. Procédé selon l'une quelconque des revendications 1 à 4, caractérisé en ce que l'on ajoute à la suspension des microcapsules réticulées, 0,5 à 2% en poids d'éthyl hydroxyéthylcellulose hydrosoluble.

6. Procédé selon l'une quelconque des revendications 1 à 5, caractérisé en ce que l'on ajoute à la suspension des microcapsules réticulées, 1 à 15% en poids de chlorure de calcium.

7. Procédé selon l'une quelconque des revendications 1 à 6, caractérisé en ce que l'on ajoute l'éthyl hydroxyéthylcellulose hydrosoluble et le chlorure de calcium en présence d'un agent tensio-actif non ionique, sous agitation, de 400 à 600 tours/minute à 20°C.

8. Procédé selon l'une quelconque des revendications 1 à 7 caractérisé en ce que le véhicule solide par lequel on traite les microcapsules réticulées est le talc, à une concentration pondérale de 7 à 13% de la suspension desdites microcapsules.

9. Procédé selon l'une quelconque des revendications 1 à 8 caractérisé en ce que la matière active à microencapsuler est un dérivé pyréthrinoïde.

10. Procédé selon l'une quelconque des revendications 1 à 9, caractérisée en ce que la matière active à microencapsuler est le (1R, cis) 2,2-diméthyl 3-(2,2-dibromovinyl) cyclopropane-1-carboxylate de (S)α-cyano 3-phénoxy benzyl ou décaméthrine.

11. Procédé selon l'une quelconque des revendications 1 à 9, caractérisé en ce que la matière active à microencapsuler est le (1R,3S,E) 2,2-diméthyl 3-(2-oxo 2,3,4,5-tétrahydro 3-thiophénylidène méthyl) cyclopropane-1-carboxylate de 5-benzyl 3-furylméthylene ou kadéthrine.

12. Procédé selon l'une quelconque des revendications 1 à 8, caractérisé en ce que la matière active à encapsuler est un élément choisi dans le groupe constitué par les virus, les enzymes, les phéromones les hormones juvéniles.

13. Procédé selon la revendication 12, caractérisé en ce que le virus est le Bacillus Thurigiensis ou l'"Heliothis Nuclear Polyhedrosis Virus".

14. Procédé selon l'une quelconque des revendications 1 à 8, caractérisé en ce que la matière active à encapsuler est un milieu nutritif artificiel.

15. Procédé selon l'une quelconque des revendications 1 à 8, caractérisé en ce que la matière active à encapsuler est un élément choisi dans le groupe constitué par les insecticides non pyréthrinoïdes, les herbicides, les biocides, les fongicides, les acaricides et les nématicides.

16. Procédé selon l'une quelconque des revendications 1 à 8, caractérisé en ce que la matière active à encapsuler est une association de deux ou plusieurs agents tels que définis à l'une quelconque des revendications 9 à 15.

**Patentansprüche**

1. Verfahren zur Herstellung von stabilen Suspensionen oder Pulvern stabiler Mikrokapseln mit einer variablen Porosität, enthaltend zumindest einen Wirkstoff, wobei das Verfahren die folgenden Stufen umfaßt:

— die Herstellung einer "Öl in Wasser"-Emulsion aus einer kolloidalen Lösung von Gelatine und Gummi arabicum bzw. Akaziengummi mit gleichem Gewicht, deren Gesamtkonzentration 4 bis 6% des Gewichts der Emulsion beträgt, und einer öligen Emulsion, Lösung oder Dispersion des oder der Wirkstoffe, wobei die Konzentration 1 bis 5% des Gewichts der "Öl in Wasser"-Emulsion beträgt, unter Rühren bei einer Temperatur bei etwa 50°C und in Gegenwart eines Emulgiermittels,

— die Koazervation und die anschließende Mikroeinkapselung der emulgierten den oder die Wirkstoffe enthaltenden Tröpfchen durch Einstellung des pH-Wertes des Reaktionsmilieus auf 4,2 bis 4,4 mit einer Säure und anschließend Absenkung der Temperatur auf 20°C unter ständigem Rühren,

— die Vernetzung der Wände der gebildeten Mikrokapseln durch die Wirkung eines Mittels, das befähigt ist, mit den Aminofunktionen der Gelatine zu reagieren,

— und die Formulierung der erhaltenen Mikrokapseln, dadurch gekennzeichnet, daß

11

**0 025 379**

man der "Öl in Wasser"-Emulsion eine Menge an organolöslicher Äthylhydroxyäthylcellulose zugibt, die entsprechend der gewünschten Porosität der Mikrokapseln variiert und die umso geringer ist, je größer die Menge an organolöslicher Äthylhydroxyäthylcellulose ist,

man mit den gebildeten Mikrokapseln nach der Koazervation nach und nach als Vernetzungsmittel Glutaraldehyd und Tanin unter langsamem Rühren bie 20°C umsetzt und die vernetzten Mikrokapseln erhält, die

man entweder unter langsamen Rühren bie 20°C mit wasserlöscliher Äthylhydroxyäthylcellulose versetzt und die konzentrierte Suspension der Mikrokapseln erhält,

oder man mit einem festen Träger unter Rühren bie 20°C behandelt, und nach dem Trocknen das gewünschte Pulver isoliert.

2. Verfahren gemäß Anspruch 1, dadurch gekennzeichnet, daß man die "Öl in Wasser"-Emulsion durch Rühren bei etwa 400 bis 600 Upm in Gegenwart eines Antischaummittels, wie eines sekundären Alkohols, wie sekundärer Octylalkohol, dessen Konzentration 0,2 bis 0,7% des Gewichts der genannten Emulsion beträgt, und in Gegenwart eines Gemisches eines anionischen oberflächenaktiven Mittels und eines nicht ionischen oberfläckenaktiven Mittels herstellt.

3. Verfahren gemäß Anspruch 1 oder 2, dadurch gekennzeichnet, daß die Konzentration an organolöslicher Äthylhydroxyäthylcellulose in der "Öl in Wasser"-Emulsion 0,01 bis 20% des Gewichts der Kolloide beträgt.

4. Verfahren gemäß einem der Ansprüche 1 bis 3, dadurch gekennzeichnet, daß die Konzentration des Glutaraldehyds 2 bis 5% und diejenige des Tanins 3 bis 8% des Gewichts der Kolloide beträgt, und daß das Rühren in diesem Stadium mit etwa 400 bis 600 Upm bei 20°C erfolgt.

5. Verfahren gemäß einem der Ansprüche 1 bis 4, dadurch gekennzeichnet, daß man zu der Suspension der vernetzten Mikrokapseln 0,5 bis 2 Gew.-% wasserlösliche Äthylhydroxyäthylcellulose zugibt.

6. Verfahren gemäß einem der Ansprüche 1 bis 5, dadurch gekennzeichnet, daß man zu der Suspension der vernetzten Mikrokapseln 1 bis 15 Gew.-% Calciumchlorid zugibt.

7. Verfahren gemäß einem der Ansprüche 1 bis 6, dadurch gekennzeichnet, daß man wasserlösliche Äthylhydroxyäthylcellulose und Calciumchlorid in Gegenwart eines nicht ionischen oberflächenaktiven Mittels, unter Rühren bie 400 bis 600 Upm bei 20°C zugibt.

8. Verfahren gemäß einem der Ansprüche 1 bis 7, dadurch gekennzeichet, daß der feste Träger, mit dem man die vernetzten Mikrokapseln behandelt, Talk mit einer Gewichtskonzentration von 7 bis 13% der Suspension der Mikrokapseln ist.

9. Verfahren gemäß einen der Ansprüche 1 bis 8, dadurch gekennzeichnet, daß der einzukapselnde Wirkstoff ein Pyrethrinoidderivat ist.

10. Verfahren gemäß einem der Ansprüche 1 bis 9, dadurch gekennzeichnet, daß der einzukapselnde Wirkstoff (S)-$\alpha$-Cyano-3-phenoxy-benzyl-(1R,cis)-2,2-dimethyl-3-(2,2-dibromvinyl)-cyclopropan-1-carboxylat oder Decamethrin ist.

11. Verfahren gemäß einem der Ansprüche 1 bis 9, dadurch gekennzeichnet, daß der einzukapselnde Wirkstoff 5-Benzyl-3-furylmethyl-(1R,3S,E)-2,2-dimethyl-3-(2-oxo-2,3,4,5-tetrahydro-3-thiophenyliden-methyl)-cyclopropan-1-carboxylat oder Kadethrin ist.

12. Verfahren gemäß einem der Ansprüche 1 bis 8, dadurch gekennzeichnet, daß der einzukapselnde Wirkstoff ein Bestandteil, ausgewählt unter den Viren, den Enzymen, den Pheromonen, und den Juvenilhormonen, ist.

13. Verfahren gemäß Anspruch 12, dadurch gekennzeichnet, daß der Virus Bacillus Thurigiensis oder "Heliothis Nuclear Polyhedrosis Virus" ist.

14. Verfahren gemäß einem der Ansprüche 1 bis 8, dadurch gekennzeichnet, daß der einzukapselnde Wirkstoff ein künstliches Nährmilieu ist.

15. Verfahren gemäß einem der Ansprüche 1 bis 8, dadurch gekennzeichnet, daß der einzukapselnde Wirkstoff ein Bestandteil ist, ausgewählt unter den nicht pyrethrinoiden Insektiziden, den Herbiziden, den Bioziden, den Fungiziden, den Akariziden und den Nematiziden.

16. Verfahren gemäß einem der Ansprüche 1 bis 8, dadurch gekennzeichnet, daß der einzukapselnde Wirkstoff eine Assoziation von 2 oder mehreren Mitteln, gemäß einem der Ansprüche 9 bis 15, ist.

**Claims**

1. Preparation process for stable suspensions or powders of microcapsules which are stable and of variable porosity, containing at least one active material, which process comprises the following stages:

— preparation of an "oil in water" emulsion, starting with a colloidal solution of gelatine and gum arabic of equal weight, their total concentration being from 4 to 6% of the weight of the emulsion and of an emulsion, a solution or an oily dispersion of the active material or materials, the concentration being from 1 to 5% of the weight of the "oil in water" emulsion, under agitation and at a temperature close to 50°C and in the presence of an emulsifying means,
— coacervation and consecutive micro-encapsulation of emulsified droplets containing active material(s) by adjustment of the pH value of the reactional medium to 4.2—4.4 by means of an acid then by lowering the temperature to about 20°C, still under agitation,
— reticulation of the walls of the microcapsules formed by action of a means able to react with the amine functions of the gelatin,
— and formulation of the microcapsules obtained, process characterized in that:
— a quantity of organosoluble ethyl hydroxyethylcellulose, variable according to the desired porosity of the microcapsules, is added to the said "oil in water" emulsion, the porosity being that much less as the quantity of organosoluble ethyl hydroxyethylcellulose is greater,
— following successive coacervation, glutaric aldehyde and tannin, as reticulation means, are made to act on the microcapsules formed, under mild agitation at 20°C and reticulated microcapsules are obtained,

either to which under mild agitation at 20°C, hydrosoluble ethyl hydroxyethylcellulose is added and a concentrated suspension of microcapsules is obtained,
or which are treated with a solid vehicle, under agitation at 20°C and, after drying, the powder sought is isolated.

2. Process according to claim 1, characterized in that the "oil in water" emulsion is prepared by agitation of about 400—600 turns/minute, in the presence of an anti-frothing agent, such as a secondary alcohol, like secondary octyl alcohol, the concentration of which is 0.2 to 0.7% of the weight of the said emulsion and in the presence of a mixture of an anionic surface-active agent and a non-ionic surface-active agent.

3. Process according to Claim 1 or 2, characterized in that the concentration of organosoluble ethyl hydroxyethylcellulose in the "oil in water" emulsion is 0.01 to 20% of the weight of the colloids.

4. Process according to any one of Claims 1—3, characterized in that the concentration of glutaric aldehyde is 2 to 5% and that of tannin is 3 to 8% of the weight of the colloids and the agitation, at this stage, is about 400 to 600 turns/minutes at 20°C.

5. Process according to any one of Claims 1—4, characterized in that 0.5 to 2% by weight of hydrosoluble ethyl hydroxyethylcellulose is added to the suspension of reticulated microcapsules.

6. Process according to any one of Claims 1—5, characterized in that 1 to 15% by weight of calcium chloride is added to the suspension of reticulated microcapsules.

7. Process according to any one of Claims 1—6, characterized in that the hydrosoluble ethyl hydroxyethylcellulose and the calcium chloride are added in the presence of a non-ionic surface-active agent, under agitation of 400—600 turns/minute at 20°C.

8. Process according to any one of Claims 1—7, characterized in that the solid vehicle with which the reticulated microcapsules are treated is talc, at a concentration of 7 to 13% by weight of the suspension of the said microcapsules.

9. Process according to any one of Claims 1—8, characterized in that the active material for microencapsulation is a pyrethrinoid derivative.

10. Process according to any one of Claims 1—9, characterized in that the active material for microencapsulation is (1R,cis) 2,2-dimethyl-3-(2,2-dibromovinyl)cyclopropane-1-carboxylate of (S) α-cyano-3-phenoxybenzyl or decamethrin.

11. Process according to any one of Claims 1—9, characterized in that the active material for microencapsulation is (1R,3S,E) 2,2-dimethyl-3-(2-oxo-2,3,4,5-tetrahydro-3-thiophenylidene-methyl)cyclopropane-1-carboxylate of 5-benzyl-3-furylmethyl or kadethrin.

12. Process according to any one of Claims 1—8, characterized in that the active material for encapsulation is chosen from the group consistuted by viruses, enzymes, pheromones, and juvenile hormones.

13. Process according to Claim 12, characterized in that the virus is Bacillus Thurigiensis or "Heliothis Nuclear Polyhedrosis Virus".

14. Process according to any one of Claims 1—8, characterized in that the active material for encapsulation is an artificial nutritive medium.

15. Process according to any one of Claims 1—8, characterized in that the active material for encapsulation is an element chosen from the group constituted by non-pyrethrinoid insecticides, herbicides, biocides, fungicides, acaricides and nematocides.

16. Process according to any one of Claims 1—8, characterized in that the active material for encapsulation is a combination of two or more agents as defined in any one of the Claims 9—15.